(19) Europäisches Patentamt
European Patent Office
Office européen des brevets

(11) **EP 3 765 441 B1**

(12) **FASCICULE DE BREVET EUROPEEN**

(45) Date de publication et mention
de la délivrance du brevet:
**27.03.2024 Bulletin 2024/13**

(21) Numéro de dépôt: **19717535.9**

(22) Date de dépôt: **14.03.2019**

(51) Classification Internationale des Brevets (IPC):
***C07C 253/30*** *(2006.01)*    ***C07C 255/65*** *(2006.01)*

(52) Classification Coopérative des Brevets (CPC):
(C-Sets disponibles)
**C07C 253/30;** C07C 2601/14         (Cont.)

(86) Numéro de dépôt international:
**PCT/FR2019/050565**

(87) Numéro de publication internationale:
**WO 2019/175510 (19.09.2019 Gazette 2019/38)**

(54) **PROCÉDÉ DE SYNTHÈSE DE COMPOSÉS AZOÏQUES**

VERFAHREN ZUR SYNTHESE VON AZOVERBINDUNGEN

PROCESS FOR SYNTHESIZING AZO COMPOUNDS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **16.03.2018 FR 1852264**

(43) Date de publication de la demande:
**20.01.2021 Bulletin 2021/03**

(73) Titulaire: **ARKEMA FRANCE**
**92700 Colombes (FR)**

(72) Inventeurs:
• **SAGE, Jean-Marc**
**69491 PIERRE-BENITE CEDEX (FR)**
• **BOSSOUTROT, Jean-Michel**
**69491 PIERRE-BENITE CEDEX (FR)**

(74) Mandataire: **Arkema Patent**
**Arkema France**
**DRD-DPI**
**420, rue d'Estienne d'Orves**
**92705 Colombes Cedex (FR)**

(56) Documents cités:
**EP-A1- 2 821 393    EP-A1- 3 098 228**

• **SERGIENKO ET AL: "Specific structural features
of molybdenum (VI) and tungsten (VI) oxo
complexes with
1-hydroxyethylidenediphosphonic acid",
CRYSTALLOGRAPHY REPORTS, NAUKA
/INTERPERIODICA, MOSCOW, RU, vol. 44, no. 5,
1999, pages 877-893, XP009509129, ISSN:
1063-7745**

**(Cont. page suivante)**

(52) Classification Coopérative des Brevets (CPC):
(Cont.)

C-Sets
**C07C 253/30, C07C 255/65**

**Description**

La présente invention concerne un procédé de synthèse d'un composé azoïque, tel que l'azo-bis-isobutyronitrile (AZDN ou AIBN), par oxydation d'un composé hydrazo.

L'AZDN est un composé azoïque couramment utilisé dans les procédés de polymérisation radicalaire, notamment en tant qu'amorceur ou catalyseur. Il est également connu comme agent gonflant pour la fabrication de mousses en PVC ou de joints en silicone.

Les composés azoïques sont classiquement produits par l'oxydation au chlore d'un composé hydrazo (WO 2006/067315). Ce procédé a également été appliqué à d'autres composés azoïques tels que les hydrazoamides (GB 976552). Cependant, ce procédé de synthèse a pour inconvénient majeur, outre la dangerosité intrinsèque du chlore, de générer de l'acide chlorhydrique comme sous-produit, de sorte que les effluents ne peuvent pas être facilement recyclés. Il en ressort que le procédé au chlore n'est donc pas adapté aux contraintes environnementales actuelles.

Pour surmonter cet inconvénient, il a été proposé un procédé de synthèse de composés azoïques utilisant un agent oxydant dépourvu de chlore tel que le peroxyde d'hydrogène (EP2821393). Afin que la réaction soit suffisamment complète et rapide, ce procédé nécessite la présence d'un agent activant, généralement un composé du brome tel qu'un bromure, ou encore un dérivé d'iode, utilisé en milieu acide. La réactivité du peroxyde d'hydrogène, et ainsi le rendement de la réaction, sont généralement améliorés par l'emploi de catalyseurs métalliques en plus des composés de brome ou d'iode, en particulier de catalyseurs à base de molybdène ou de tungstène (cf. CS232350 et A. PALOMO et coll., « Afinidad », (1985), 42(397), 312-314) qui ont l'avantage d'être moins toxiques que le tellure, le vanadium ou le sélénium, par exemple.

En dépit de ses avantages incontestables par rapport au procédé au chlore, le procédé au peroxyde d'hydrogène génère toutefois lui aussi des effluents potentiellement dommageables pour l'environnement, à savoir les eaux résiduelles (aussi appelées eaux-mères) résultant de la filtration du milieu réactionnel pour en séparer l'AZDN. Ces eaux résiduelles contiennent en effet une quantité non négligeable du catalyseur et des activateurs utilisés. Il a donc été suggéré, non seulement pour des questions environnementales, mais également pour améliorer l'économie du procédé, de recycler ces eaux résiduelles dans la réaction, éventuellement après concentration (cf. par exemple CS 239407 et CS 237123).

En outre, le procédé de synthèse conduit également à la formation d'ions ammonium. Or, en présence de ces ions ammonium, le catalyseur a tendance à précipiter sous forme d'un dépôt jaune qui pollue le composé azoïque formé et, par conséquent, diminue la pureté de ce dernier.

Par ailleurs, la précipitation du catalyseur provoque également l'instabilité des eaux résiduelles, limitant ainsi leur recyclage. Aussi, la demande EP2821393 propose-telle un procédé dans lequel l'ajout, dans le milieu réactionnel, d'un agent réducteur tel que l'hydrazine permet d'améliorer la stabilité des eaux résiduelles. Cependant, cette demande ne résout pas complètement les problèmes de stabilité du catalyseur, notamment lorsque les eaux résiduelles sont concentrées avant d'être recyclées. En effet, on constate, après plusieurs recyclages, la précipitation d'une espèce contenant des ions ammonium alors qu'aucun ion ammonium n'a été introduit dans le milieu. Ces ions ammonium, accumulés au cours du procédé et issus de la dégradation des fonctions nitriles du dérivé azoïque utilisé, induisent une précipitation du catalyseur.

Il convient donc de trouver un procédé de synthèse de composés azoïques dans lequel notamment le catalyseur ne précipite pas, conduisant ainsi à la synthèse d'un composé azoïque au sein duquel le taux de catalyseur résiduel est fortement diminué et à une meilleure stabilité des eaux résiduelles.

Ainsi la présente invention se propose de résoudre le problème de précipitation du catalyseur par l'ajout d'un agent complexant particulier qui sera décrit dans la description qui suit. D'autres objectifs apparaîtront également dans ladite description de la présente invention.

Selon un premier objet, l'invention concerne un procédé de synthèse d'un composé azoïque, ledit procédé comprenant les étapes :

a) faire réagir un agent oxydant avec un composé hydrazo, au moins un catalyseur et au moins un composé de formule (I) :

$$(R_1)(R_2)C(PO_3(R_3)_2)_2 \qquad (I)$$

dans laquelle

R$_1$ et R$_2$, identiques ou différents, sont choisis indépendamment l'un de l'autre parmi l'atome d'hydrogène, une chaîne hydrocarbonée saturée ou insaturée, linéaire, ramifiée ou cyclique, éventuellement substituée, -OH et -O-alkyle, où « alkyle » représente une chaîne saturée hydrocarbonée, éventuellement substituée, linéaire ou ramifiée, comportant de 1 à 6 atomes de carbone ; et
R$_3$ est choisi parmi l'atome d'hydrogène et les ions métalliques ou ammoniums ;

de manière à former une solution renfermant un composé azoïque ;

b) récupérer tout ou partie du mélange réactionnel obtenu à l'étape a) ;
c) séparer le mélange réactionnel récupéré en une fraction contenant le composé azoïque et une fraction d'eaux résiduelles ; et
d) récupérer et éventuellement laver et sécher, le composé azoïque obtenu.

Dans le procédé ci-dessus, le composé de formule (I) agit comme agent complexant du catalyseur et sera désigné, dans le reste de la demande par le terme « agent complexant ».

Par « éventuellement substitué », on entend que la chaîne hydrocarbonée et le radical alkyle peuvent être substitués par -OH, un halogène et $N(R_4)(R_5)$, $R_4$ et $R_5$, identiques ou différents, sont choisis indépendamment l'un de l'autre parmi l'atome d'hydrogène, un radical alkyle, linéaire ou ramifié, comportant de 1 à 6 atomes de carbone ou bien $R_4$ et $R_5$, peuvent former ensemble, et avec l'atome d'azote auquel ils sont rattachés, un cycle à 4, 5, 6 ou 7 chaînons, de préférence à 5 ou 6 chaînons.

Selon un mode de réalisation préféré, au sein de la formule (I), $R_1$ est $-CH_3$ et $R_2$ est -OH.

Dans la formule (I), $R_3$ est choisi parmi -H et les ions métalliques ou ammoniums. Lesdits ions métalliques peuvent être choisis, de manière non limitative, parmi les métaux représentés par les colonnes 1 à 13 du tableau périodique des éléments, de préférence parmi le sodium, le lithium, le potassium, le calcium, le magnésium, l'aluminium, le zinc, le cadmium, le manganèse, le nickel, le cobalt, le cérium, le cuivre, l'étain, le fer et le chrome, de préférence encore parmi le sodium, le lithium, le potassium, le calcium, le magnésium, tout particulièrement parmi le sodium et le potassium. $R_3$ peut également être choisi parmi les ions ammonium et les ions alkyl ammonium, en particulier les alkyl amines, les alkylène-amines et les alcanolamines ne contenant pas plus de deux groupes amine, tels que l'éthylamine, la diéthylamine, la propylamine, l'hexylamine, la 2-éthylhexylamine, la N-butyléthanolamine et les mono-, di- ou tri-éthanolamines.

Au sein de la formule (I), les radicaux $R_3$ peuvent être identiques ou différents. Selon un mode de réalisation préféré, ils sont tous identiques, et de manière tout particulièrement préférée, ils représentent tous -H.

Dans un mode de réalisation préféré, l'agent complexant peut être choisi parmi les composés alkyldiphosphoniques, tels que, par exemple, l'acide hydroxyméthylène diphosphonique, l'acide 1-hydroxyléthylidène-1,1-diphosphonique (HEDP) et l'acide 1-hydroxybutanediphosphonique, sous leur forme acide ou sous forme de leurs sels. De manière particulièrement préférée, l'agent complexant organique est l'acide 1-hydroxyléthylidène-1,1-diphosphonique (HEDP) également connu sous la dénomination commerciale Dequest® 2010.

L'agent complexant peut également être l'acide zolédronique ou un de ses sels (zolédronate), c'est-à-dire l'acide 1-hydroxy-1-(1H-imidazol-1-yl)éthane-1,1-diyl]diphosphonique ou un de ses sels respectivement, ceux-ci étant commercialisés par la société NOVARTIS sous les marques Zomate®, Zomera®, Aclasta® et Reclast®.

Selon un mode de réalisation de l'invention, la masse molaire de l'agent complexant est inférieure à 500 g.mol$^{-1}$, de préférence inférieure à 450 g.mol$^{-1}$, plus particulièrement inférieure à 400 g.mol$^{-1}$.

Selon un autre mode de réalisation, l'agent complexant est soluble en milieu aqueux ou hydro-organique, de préférence en milieu aqueux, et plus particulièrement dans l'eau.

L'agent complexant peut être utilisé dans un rapport molaire de l'agent complexant aux moles de molybdène apportées par le catalyseur supérieur à 0,05:1, de préférence compris entre 0,1:1 et 5:1, plus particulièrement entre 0,5:1 et 2:1.

Le composé hydrazo oxydé lors l'étape a) du procédé peut être choisi parmi les composés hydrazo symétriques porteurs de fonctions azotées, en particulier de fonctions nitriles ou amides, telles que le 2,2'-hydrazo-bis-isobutyronitrile, le 2,2'-hydrazo-bis-méthylbutyronitrile, le 1,1'-hydrazo-bis-cyclohexanecarbonitrile ou le 2,2'-hydrazo-dicarbonamide, de préférence le 2,2'-hydrazo-bis-isobutyronitrile.

L'agent oxydant présent à l'étape a) du procédé peut être de tout type, et peut être choisi parmi les dérivés peroxydiques inorganiques tel que par exemple le peroxyde d'hydrogène, les persulfates de potassium ou de sodium, les monohydrogénopersulfates de potassium ou de sodium, le dioxygène, les dérivés organiques peroxydiques solubles dans l'eau tel par exemple l'acide peracétique. Selon un mode de réalisation préféré, l'agent oxydant est le peroxyde d'hydrogène.

L'agent oxydant est généralement introduit dans la solution aqueuse à une température de 0°C à 40°C, de préférence de 0°C à 20°C, pendant une durée allant de 2 heures à 6 heures.

L'agent oxydant est généralement utilisé en léger excès molaire par rapport au composé hydrazo. Le rapport molaire de l'agent oxydant au composé hydrazo est ainsi avantageusement compris entre 1:1 et 1,1:1, de préférence entre 1,01:1 et 1,05:1, bornes incluses.

Le catalyseur présent lors de l'étape a) du procédé comprend un composé soluble dans l'eau choisi parmi les sels et acides à base d'un métal catalytique des colonnes 5 et 6 du tableau périodique des éléments et de préférence choisi parmi le molybdène et le tungstène, de préférence le molybdène. Des exemples de tels composés solubles dans l'eau sont notamment les sels de métal alcalin ou d'ammonium de molybdène, les sels de métal alcalin ou d'ammonium de tungstène, l'acide phosphomolybdique et ses sels alcalins ou d'ammonium, l'acide phosphotungstique et ses sels alcalins ou d'ammonium, les molybdosulfates et leurs mélanges. L'acide phosphomolybdique et ses sels alcalins sont préférés, en particulier le phosphomolybdate de sodium et le phosphomolybdate de potassium.

Le catalyseur peut être utilisé dans un rapport molaire des moles de métal apportées par le catalyseur au composé hydrazo allant par exemple de 0,005:1 à 0,5:1, de préférence de 0,03:1 à 0,1:1.

Dans le procédé selon l'invention, l'agent complexant conduit à une faible précipitation du catalyseur, voire une absence de précipitation. En effet, il a été découvert de manière surprenante, que l'agent complexant de formule (I), en formant un complexe avec le catalyseur, conduit à l'absence de précipitation dudit catalyseur. Un autre avantage de l'agent complexant est qu'il permet également au catalyseur de ne pas perdre sa réactivité, ce qui permet audit catalyseur d'être recyclé et réutilisé dans le procédé sans avoir perdu son efficacité. Le complexe agent complexant/catalyseur est éliminé dans les eaux résiduelles permettant ainsi que la totalité, ou presque, du catalyseur soit récupérée dans les eaux résiduelles sans pour autant altérer la stabilité desdites eaux résiduelles.

Selon un mode de réalisation préféré, la solution à l'étape a) est une solution acide, dont le pH est de préférence compris entre 0 et 2. Ainsi, pour que le pH soit acide, il est possible d'ajouter un acide organique ou inorganique. De manière préférée, si l'acide est un acide inorganique, il est choisi parmi l'acide chlorhydrique, l'acide sulfurique, l'acide bromhydrique, l'acide phosphorique, et leurs mélanges, de préférence l'acide chlorhydrique. De façon préférée, si l'acide est un acide organique, il est choisi parmi l'acide formique, l'acide acétique, et leurs mélanges. L'acide peut être utilisé dans un rapport molaire de l'acide au composé hydrazo allant par exemple de 1:1 à 1:5.

Pour encore améliorer la cinétique de la réaction et/ou le rendement réactionnel, il est avantageux d'opérer l'étape a) d'oxydation en présence d'un agent activant. Ledit agent activant peut être choisi parmi les halogènes et les halogénures, tels que par exemple, et de manière non limitative, le brome, l'iode, les acides bromhydrique et iodhydrique et leurs sels, de préférence choisi parmi l'acide bromhydrique et un bromure de métal alcalin tels que par exemple, le bromure de potassium et le bromure de sodium. Il peut être utilisé dans un rapport molaire de l'activateur au composé hydrazo allant par exemple de 0,1:1 à 1:1, plus particulièrement de 0,3:1 à 0,7:1.

Un tensioactif peut éventuellement être ajouté à la solution, notamment un tensioactif anionique, en particulier un sulfosuccinate d'alkyle tel que le sulfosuccinate de di-2-éthylhexyle.

Au cours de l'étape a) d'oxydation, l'ordre d'introduction des différents composés dans cette solution n'est pas critique.

À l'issue de cette étape a), on obtient une solution aqueuse renfermant un composé azoïque, au moins un complexe agent complexant /catalyseur et une quantité d'agent oxydant. Ladite solution aqueuse peut éventuellement comprendre une quantité d'un acide organique ou inorganique et/ou d'un agent activant, et/ou d'un tensioactif si ces composés ont été ajoutés au cours de l'étape a).

Il peut être avantageux d'ajouter à la solution aqueuse obtenue à l'issue de l'étape a) au moins un agent réducteur choisi parmi l'hydrazine, le sulfite de sodium, le bisulfite de sodium et leurs mélanges. Selon un mode de réalisation préféré, et dans le cas où le composé soluble dans l'eau est l'acide phosphomolybdique, l'agent réducteur est l'hydrazine.

L'hydrazine a l'avantage de ne former que de l'eau et de l'azote gazeux dans les effluents et dans la boucle de recyclage du procédé, ce qui évite l'accumulation de sels au cours du recyclage.

Le mélange réactionnel alors obtenu à l'issue de l'étape a) est ensuite récupéré en totalité ou partie, par exemple à hauteur de 30% à 60% en poids, notamment de 45% à 55% en poids, au cours de l'étape b) du procédé selon l'invention.

Le mélange réactionnel éventuellement non récupéré à l'issue de l'étape b) peut être recyclé dans l'étape a), tandis que le mélange réactionnel récupéré est séparé, dans l'étape c), en une fraction contenant le composé azoïque et une fraction d'eaux résiduelles. Au cours de l'étape c), le composé azoïque peut être séparé par toute technique connue de l'homme du métier, de préférence par filtration ou centrifugation, et plus particulièrement par centrifugation.

Il peut être avantageux d'ajouter à la fraction d'eaux résiduelles au moins un agent réducteur (même s'il en a déjà été ajouté à l'issue de l'étape a)). Ledit agent réducteur est choisi parmi ceux déjà décrits plus haut, et par exemple parmi l'hydrazine, le sulfite de sodium, le bisulfite de sodium et leurs mélanges.

Selon un mode de réalisation préféré, et dans le cas où le composé soluble dans l'eau est l'acide phosphomolybdique, l'agent réducteur est l'hydrazine. Selon un mode de réalisation préféré, l'agent réducteur est ajouté à l'issue de l'étape c). Selon une autre mode de réalisation préféré de l'invention, l'agent réducteur est ajouté à l'issue de l'étape a) et à l'issue de l'étape c).

L'agent réducteur est généralement utilisé en quantité nécessaire et suffisante pour neutraliser l'excès d'agent oxydant mesuré à l'issue de l'étape a) ou de l'étape c) du procédé.

La fraction contenant le composé azoïque peut être lavée une ou plusieurs fois à l'eau, dans une étape d), de façon à récupérer un composé azoïque ayant une pureté supérieure à 90% et des eaux de lavage. Ce haut degré de pureté du composé azoïque est notamment obtenu par l'absence de précipitation du catalyseur, cette absence de précipitation étant liée à la présence de l'agent complexant qui, comme décrit précédemment, forme un complexe avec le catalyseur, ledit complexe étant éliminé dans les eaux résiduelles.

Le composé azoïque récupéré après l'étape de séparation c) ou l'étape de lavage d) peut éventuellement subir une étape supplémentaire de purification, par exemple, par recristallisation. Cette recristallisation peut être effectuée par toute technique connue de l'homme du métier telle que, par exemple, celle décrite dans la demande de brevet WO 2006/072699 qui précise que la recristallisation peut être effectuée dans un solvant tels que par exemple le chlorure de méthylène, le méthanol et la méthyléthylcétone. Cette étape permet d'éliminer toute trace éventuelle d'agent complexant.

Dans une étape ultérieure e) du procédé, la fraction d'eaux résiduelles peut être recyclée en tout ou partie dans l'étape a), étant entendu que les étapes a) à e) sont éventuellement répétées au moins une fois, c'est-à-dire que la fraction d'eaux résiduelles peut être recyclée au moins deux fois. Dans une variante du procédé selon l'invention, la fraction d'eaux résiduelles peut être concentrée, notamment par distillation, avant d'être recyclée. Le distillat obtenu peut alors être facilement traité, par exemple incinéré, en vue d'être rejeté dans l'environnement.

Le procédé selon l'invention permet, entre autres, un traitement aisé des effluents en vue de leur recyclage dans le procédé et/ou de leur traitement en vue de leur rejet dans l'environnement. À cette fin, il peut comprendre une étape supplémentaire de traitement de tout ou partie de la fraction d'eaux résiduelles produites dans l'étape c) et/ou des eaux de lavage produites dans l'étape d) à l'aide d'un adsorbant, tel que du charbon actif, afin de retenir le métal catalytique.

Le procédé selon l'invention peut en outre comprendre une étape de récupération du métal catalytique sous forme de solution aqueuse, par traitement de l'adsorbant à l'aide d'une solution aqueuse basique, notamment d'hydroxyde de sodium. On préfère industriellement faire passer la fraction d'eaux résiduelles et/ou des eaux de lavage à traiter sur une colonne contenant l'adsorbant, sous une forme par exemple granulée, puis récupérer le métal catalytique par passage d'une solution basique sur cette colonne, selon les techniques bien connues d'utilisation de ces adsorbants.

La solution aqueuse de métal catalytique peut ainsi être recyclée dans l'étape a) du procédé, éventuellement après concentration, tandis que les eaux résiduelles résultant de la filtration de l'adsorbant et/ou les eaux de lavage de l'adsorbant peuvent être rejetées dans l'environnement. Ceci permet de récupérer et recycler une quantité efficace de catalyseur, tout en rejetant dans l'environnement un effluent très pauvre en catalyseur.

Le procédé selon l'invention permet d'obtenir en un temps raisonnable une granulométrie élevée des composés azoïques produits, typiquement voisine de 150 $\mu$m (telle que mesurée par diffraction laser), qui peut s'avérer avantageuse dans certaines applications.

Ainsi, le procédé de la présente invention est tout particulièrement avantageux lorsque les eaux résiduelles et/ou le catalyseur et/ou l'agent activateur, et en particulier les eaux résiduelles et/ou tous les composants du système catalytique, sont recyclés. Ces opérations de recyclage présentent en autres le grand avantage de ne pas rejeter dans les effluents le système catalytique et en particulier les sels métalliques onéreux, mais aussi et surtout néfastes pour l'environnement.

L'invention a également pour objet l'utilisation dudit composé de formule (I) en tant qu'agent complexant d'un catalyseur, par exemple d'un catalyseur tel que défini ci-dessus, dans un procédé de synthèse d'un composé azoïque comprenant une étape faisant réagir un agent oxydant avec un composé hydrazo, au moins un catalyseur et au moins ledit composé de formule (I) de manière à former une solution renfermant un composé azoïque. De préférence, ledit composé de formule (I) est utilisé en tant qu'agent complexant d'un catalyseur, par exemple d'un catalyseur tel que défini ci-dessus, dans un procédé de synthèse d'un composé azoïque, par exemple dans le procédé tel que défini ci-dessus.

L'invention a pour troisième objet les eaux résiduelles comprenant le complexe agent complexant/catalyseur décrit précédemment, lesdites eaux résiduelles étant obtenues au cours du procédé de synthèse d'un composé azoïque selon l'invention.

L'invention sera mieux comprise à la lumière des exemples suivants, qui sont donnés à titre uniquement illustratif et ne limitent en aucun cas la portée de l'invention.

## EXEMPLES

Dans les exemples suivants :

- DHC désigne l'hydrazo-bis-isobutyronitrile, composé hydrazo obtenu industriellement par réaction du cyanhydrine d'acétone sur l'hydrate d'hydrazine, filtration puis lavage à l'eau, et conservé au réfrigérateur (T < 10°C). Son taux d'humidité est de 12,7% et sa pureté est supérieure à 99% par analyse.
- l'acide phosphomolybdique utilisé est un produit vendu par la société Aldrich qui correspond à la formule $H_3[P(Mo_3O_{10})_4].xH_2O$ et est utilisé comme tel (masse molaire 1825,25 g/mol sous forme anhydre). Le produit utilisé possède une teneur de 50% en molybdène.
- DOSS désigne le sulfosuccinate de di(2-éthylhexyle).
- AZDN (ou AIBN) désigne l'azo-bis-isobutyronitrile.

*Méthode de prélèvement et de dosage du taux de peroxyde résiduel*

Dans les exemples suivants, les taux de peroxyde résiduel sont mesurés comme suit. On prélève environ 3 mL à 5 mL du mélange réactionnel en suspension que l'on filtre afin d'éliminer le DHC et l'AZDN solide présents. On pèse environ exactement 1 gramme de la solution filtrée que l'on introduit dans un flacon de 250 mL, on rajoute 50 mL d'eau distillée, 15 mL d'acide sulfurique à 30% en poids et 15 mL d'une solution de KI à 30%. Le flacon est bouché puis laissé à l'obscurité pendant 15 mn. On titre ensuite avec une solution de thiosulfate de sodium de normalité 0,1 N jusqu'à disparition de la coloration jaune. La teneur en peroxyde d'hydrogène ($H_2O_2$) résiduel est calculée comme suit :

$$\% \; H_2O_2 \; \text{résiduel} \; = \; \frac{\text{Volume de thiosulfate de sodium 0,1 N (en mL)}}{2 \times 100 \times \; \text{masse de filtrat (en grammes)}}$$

Le dosage du molybdène résiduel est effectué par la technique d'ICP Optique (ou Plasma Couplé Induction). La mesure de granulométrie est effectuée à l'aide d'un appareil Masterziser® S. La mesure se fait sur les cristaux humides en utilisant comme dispersant de l'eau et une goutte de tensioactif Igepal® (nonylphénol éthoxylé), après 10 minutes de circulation dans la cellule de mesure.

### Exemple 1 : Test d'agents complexants

On prépare un mélange contenant 100 mL d'une solution aqueuse de bromure d'hydrogène à 5% en masse, 1,5 g d'acide phosphomolybdique (catalyseur) et 1 g d'agent complexant.

Au bout de 30 minutes, on effectue une première observation sur la solubilité de l'acide phosphomolybdique en absence de bromure d'ammonium ($NH_4Br$).

On ajoute ensuite 10 mL d'une solution aqueuse à 37,5% de bromure d'ammonium. Au bout de 30 minutes, une seconde observation est effectuée sur la solubilité de l'acide phosphomolybdique en présence de bromure d'ammonium.

Les résultats sont rassemblés dans le Tableau 1 suivant :

-- Tableau 1 --

| *Agent complexant* | *Présence de précipité (sans $NH_4Br$)* | *Présence de précipité (avec $NH_4Br$)* |
|---|---|---|
| Acide 1-hydroxyéthylidène-1,1-diphosphonique (Dequest® 2010) | NON | NON |
| Acide amino-triméthylène phosphonique (Dequest® 2000) | OUI | OUI |
| Acide diéthylènetriamine penta(méthylène phosphonique) (Dequest® 2060) | OUI | OUI |
| 2,2'-éthylènebis(nitrilométhylidène)diphénol N,N'-éthylènebis(salicylimine) (Salen) | OUI | OUI |
| Glycérol | NON | OUI |
| Acide 2-amino-3-(1H-imidazol-4-yl)propanoïque (DL Histidine) | OUI | OUI |
| Acide 2-amino-3-mercapto-propanoïque (DL Cystéine) | NON | OUI |
| Méthionine | NON | OUI |
| 1,10-phénantroline | OUI | OUI |
| Blanc | NON | OUI |

Les tests démontrent que les composés autres que ceux répondant à la formule (I) ne permettent pas d'éviter l'apparition d'un précipité en présence d'ions ammonium. En revanche, lorsque l'agent complexant répond à la définition de la formule (I), aucun précipité n'est observé. Cette absence de précipité traduit la stabilité de l'acide phosphomolybdique en présence de l'agent complexant, dans un milieu renfermant des ions ammonium. L'agent complexant de formule (I), par exemple le Dequest® 2010, permet d'éviter la précipitation de l'acide phosphomolybdique en présence d'ions ammonium.

Un autre test, réalisé dans les même conditions opératoires que précédemment, a été effectué afin de déterminer la quantité de composé de formule (I) suffisante pour éviter la précipitaton de l'acide phosphomolybdique en présence de $NH_4Br$ : la quantité d'agent complexant de formule (I) a été progressivement diminuée jusqu'à observer la formation d'un précipité.

Cette série de tests a permis de démontrer que, dans une solution comprenant contenant 100 ml d'une solution aqueuse de bromure d'hydrogène à 5% en masse, 1,5 g d'acide phosphomolybdique (catalyseur) et 5 mL d'une solution $NH_4Br$ à 37,5% en masse, une quantité de 0,35 g d'agent complexant de formule (I), tel que le Dequest® 2010, est suffisante pour empêcher la précipitation de l'acide phosphomolybdique, soit un ratio molaire agent complexant/acide phosphomolybdique de 0,2.

**Exemple 2 : Contre-exemple (procédé en absence d'agent complexant)**

Dans un réacteur d'une capacité de 1,5 L et muni d'un système d'agitation permettant le brassage d'une suspension, on introduit 126 g d'AZDN (0,77 mole), 128 g de DHC (hydrazo-bis-isobutyronitrile) (0,77 mole), une solution aqueuse composée de 635 g d'eau, 35 g d'HBr, 7 g d'acide phosphomolybdique (teneur en Mo de 50%, soit 0,036 mole de molybdène), et 0,1 g de DOSS (sulfosuccinate de di-(2-éthylhexyle)). Après mise en route de l'agitation et du système de refroidissement par double enveloppe, on attend que la température se stabilise vers 10°C.

Une fois la température de 10°C atteinte, on introduit en continu, 78,05 g d'$H_2O_2$ à 35% (soit 0,80 mole d'$H_2O_2$) pendant 4 h. Au cours de la réaction, le milieu est maintenu à une température comprise entre 10°C et 12°C. On arrête la réaction environ 20 à 30 minutes après avoir introduit la totalité de la quantité d'$H_2O_2$. La fin de réaction est rendue visible par la formation de brome et par l'augmentation du potentiel rédox (avant l'introduction d'$H_2O_2$, le potentiel est d'environ 400 mV et en fin de réaction, c'est-à-dire après l'introduction de la totalité de l'$H_2O_2$, le potentiel est d'environ 800 mV). Le potentiel rédox est mesuré à l'aide d'une sonde rédox au Platine.

On filtre ensuite en totalité la suspension du réacteur. On obtient 290 g d'AZDN brut contenant 15% d'humidité soit un rendement de 95%. En outre, 20 mL d'eau sont également utilisés pour rincer le filtre et rajoutés aux eaux résiduelles.

Les eaux résiduelles de filtration obtenues, soit 740 g, sont neutralisées par de l'hydrate d'hydrazine afin d'éliminer l'excès peroxydique et le brome formé (par dosage on trouve 0,1% d'équivalent $H_2O_2$ résiduel). On se sert de l'électrode rédox afin de ramener le potentiel à sa valeur initiale soit environ 300 mV à 400 mV.

La moitié des eaux résiduelles, soit 374 g, est mis de côté pour être réutilisée directement à l'essai suivant, tandis que l'autre moitié subie une étape de concentration sous vide visant à éliminer environ 60% à 65% d'eau de cette fraction des eaux résiduelles. Ceci est effectué sous un vide de 250 millibars (25 kPa) entre 55°C et 60°C. Pour 325 g d'eaux résiduelles engagées on obtient ainsi 235 g d'une fraction aqueuse de distillat, et 90 g de solution concentrée résultante. Cette fraction concentrée résultante, contenant le système catalytique, peut donc être réemployée avec la fraction des eaux résiduelles non traitées pour le cycle suivant.

Une moitié de l'AZDN obtenu est lavé à l'eau quatre fois à l'aide de 200 mL d'eau à chaque fois. L'autre moitié de l'AZDN brut est gardée telle quelle (avec la fraction d'eaux-mères résiduelles contenue dans les cristaux) pour être recyclée lors du cycle suivant à l'exemple suivant.

Ainsi, pratiquement tous les composants du système catalytique sont recyclés, hormis la fraction inhérente aux pertes de transvasement et de prélèvements pour dosages ainsi que la fraction d'eau résiduelles retenue dans les cristaux de l'AZDN lavé produit.

**Exemple 3 : Contre-exemple (procédé sans agent complexant et avec recyclage)**

Dans le réacteur de l'exemple précédent on introduit 145 g de l'AZDN brut non lavé obtenu précédemment (soit 0,78 mole d'AZDN contenant environ 15 g d'eau résiduelles dans les cristaux), 128 g de DHC (hydrazo-bis-isobutyronitrile) (0,78 mole), 0,05 g de DOSS (sulfosuccinate de di-(2-éthylhexyle)), 374 g d'eaux résiduelles issues de la filtration du milieu réactionnel lors de l'essai précédent ainsi que les eaux résiduelles concentrées lors de l'essai précédent. Les bromures contenus dans les fractions d'eaux résiduelles recyclées sont dosées afin de déterminer l'appoint nécessaire en système catalytique. Afin de maintenir un milieu réactionnel de même composition que celle de l'exemple précédent on ajoute typiquement 4 g d'HBr, 0,78 g d'acide phosphomolybdique et 150 g d'eau, cette charge d'eau étant calculée en fonction des teneurs en eau des réactifs utilisés (solution aqueuse d'HBr, humidité du DHC) pour revenir à la composition initiale de l'essai précédent.

Cette eau, 150 g, permettra, dans un procédé industriel, de charger plus aisément le DHC solide sous forme d'une suspension aqueuse à partir d'un bac tampon par exemple.

La réaction est menée ensuite comme dans l'exemple précédent, en coulant de façon continue 78,05 g d'$H_2O_2$

à 35% sur une durée de 4 heures dans le milieu réactionnel maintenu entre 10°C et 12°C.

On opère ainsi en recyclant dans l'essai n+1 une moitié de l'AZDN et des eaux résiduelles obtenues et le concentrant de l'autre moitié des eaux résiduelles à l'essai n.

Les trois premières opérations de recyclages conduisent à l'obtention de produits blancs de pureté supérieure à 95% par analyse RMN. Au cours du quatrième recyclage on note un la formation d'un dépôt important de couleur jaune. Ce dépôt vient polluer l'AZDN obtenu, les lavages à l'eau ne permettent pas d'éliminer ce dépôt.

On note également que dans les eaux résiduelles obtenues après filtration le dépôt se forme encore avec le temps.

L'étape de concentration suivante a été menée sur ces eaux résiduelles, après une filtration pour éliminer ces cristaux. À l'issue de la concentration on note que ces cristaux se sont reformés dans le fond du bouilleur et polluent donc la solution concentrée des eaux résiduelles.

L'analyse de ce dépôt, par fluorescence X et diffraction X, montre qu'il s'agit d'un composé cristallin de phosphomolybdate d'ammonium, ces composés sont connus pour être très insolubles en milieu acide.

La formation d'ions ammonium dans le milieu parait attribuable à l'hydrolyse des sous-produits cyanés. Les ions ammonium s'accumulent ainsi au fur et à mesure des recyclages dans le milieu réactionnel provoquant la précipitation du catalyseur.

## Exemple 4 : Procédé en présence de Dequest® 2010 (selon l'invention)

On réalise le procédé de l'invention, selon le même mode opératoire que celui décrit dans le contre-exemple 2, mais en ajoutant 20,7 g d'une solution aqueuse à 60% de Dequest® 2010, (soit 1,65 moles de Dequest® 2010 par mole de molybdène) au milieu réactionnel avant l'introduction de la solution de peroxyde d'hydrogène.

On effectue une série de recyclages comme décrit dans le contre-exemple 3. Dans cet exemple on effectue ainsi 12 recyclages, sans noter de formation de précipité polluant l'AZDN obtenu ou précipitant dans les eaux mères ou au cours des opérations de concentration.

Les compléments en système catalytique sont effectués, tout au long du procédé comme indiqué dans le contre-exemple 3, mais dans cet exemple, pour un appoint de 4 g d'HBr, on ajoute en outre 1,4 g de Dequest® 2010 (soit 2,35 g de solution aqueuse à 60% de Dequest® 2010).

Au douzième recyclage les rendements en AZDN lavé restent supérieurs à 95%, La teneur en bromure résiduelle de l'AZDN lavé est de 80 ppm à 90 ppm et la pureté par analyse RMN est supérieure à 98%. La granulométrie reste constante et comprise entre 110 $\mu$m et 130 $\mu$m (diamètre moyen en nombre).

## Exemple 5 : Recristallisation de l'AZDN obtenu selon le procédé

L'AZDN obtenu selon l'exemple 4 à l'issue du douzième recyclage est lavé 3 fois avec 200 ml d'eau puis séché à température ambiante (taux d'humidité résiduelle 1% à 2% en poids). 20 g de cet AZDN sont alors mis en solution dans 250 ml de méthanol à 35°C. Lorsque les cristaux sont dissous, la température est abaissée à 2°C pour faire recristalliser l'AZDN. Le mélange est ensuite filtré à froid, la fraction de solvant méthanol récupérée est mise de côté pour être réutilisée lors de la prochaine recristallisation. Les cristaux d'AZDN récupérés sur le filtre sont lavés avec une portion d'eau de 50 mL puis séchés.

On recommence l'opération en prenant de nouveau 20 g de l'AZDN obtenu à l'issue du douzième recyclage et en utilisant la fraction de méthanol récupéré précédemment, et en complétant éventuellement pour compenser les pertes en solvant ou dissoudre totalement les 20 g d'AZDN engagés.

Cinq recristallisations successives ont ainsi été effectuées en réengageant à chaque fois la fraction de méthanol récupéré. La recherche de traces de molybdène par la méthode d'ICP Optique sur un appareil Spectromètre ICAP 6500, montre que tous les AZDN ainsi obtenus sont exempts de traces de Molybdène résiduel (limite de détection de 1 ppm).

## Revendications

**1.** Procédé de synthèse d'un composé azoïque, ledit procédé comprenant les étapes :

a) faire réagir un agent oxydant avec un composé hydrazo, au moins un catalyseur et au moins un composé de formule (I) :

$$(R_1)(R_2)C(PO_3(R_3)_2)_2 \qquad \text{(I)}$$

dans laquelle

$R_1$ et $R_2$, identiques ou différents, sont choisis indépendamment l'un de l'autre parmi l'atome d'hydrogène, une chaîne hydrocarbonée saturée ou insaturée, linéaire, ramifiée ou cyclique, éventuellement substituée, -OH et -O-alkyle, où « alkyle » représente une chaîne saturée hydrocarbonée, éventuellement substituée, linéaire ou ramifiée, comportant de 1 à 6 atomes de carbone ; et

$R_3$ est choisi parmi l'atome d'hydrogène et les ions métalliques ou ammoniums ;

de manière à former une solution renfermant un composé azoïque ;

b) récupérer tout ou partie du mélange réactionnel obtenu à l'étape a) ;

c) séparer le mélange réactionnel récupéré en une fraction contenant le composé azoïque et une fraction d'eaux résiduelles ; et

d) récupérer et éventuellement laver et sécher, le composé azoïque obtenu.

2. Procédé selon la revendication 1, dans lequel le composé de formule (I) est choisi parmi l'acide 1-hydroxy-1-(1*H*-imidazol-1-yl)éthane-1,1-diyl]diphosphonique ou un de ses sels, l'acide hydroxyméthylènediphosphonique, l'acide 1-hydroxyléthylidène-1,1-diphosphonique (HEDP) et l'acide 1-hydroxybutanediphosphonique, sous leur forme acide ou sous forme de leurs sels, de préférence le composé de formule (I) est l'acide 1-hydroxyléthylidène-1,1-diphosphonique.

3. Procédé selon la revendication 1 ou la revendication 2, dans lequel le composé hydrazo est choisi parmi les composés hydrazo symétriques porteurs de fonctions azotées, en particulier de fonctions nitriles ou amides, de préférence parmi le 2,2'-hydrazo-bis-isobutyronitrile, le 2,2'-hydrazo-bis-méthylbutyronitrile, le 1,1'-hydrazo-bis-cyclohexanecarbonitrile et le 2,2'-hydrazo-dicarbonamide, de préférence le composé hydrazo est le 2,2'-hydrazo-bis-isobutyronitrile.

4. Procédé selon l'une quelconque des revendications précédentes, dans lequel l'agent oxydant est choisi parmi les dérivés peroxydiques inorganiques, de préférence parmi le peroxyde d'hydrogène, les persulfates de potassium ou de sodium, les monohydrogénopersulfates de potassium ou de sodium, le dioxygène, les dérivés l'acide peracétique, de préférence de préférence encore l'agent oxydant est le peroxyde d'hydrogène.

5. Procédé selon l'une quelconque des revendications précédentes, dans lequel le catalyseur comprend un composé soluble dans l'eau choisi parmi les sels et acides à base d'un métal catalytique des colonnes 5 et 6 du tableau périodique des éléments et de préférence choisi parmi le molybdène et le tungstène, de préférence le molybdène.

6. Procédé selon la revendication 5, dans lequel le composé soluble est choisi parmi les sels de métal alcalin ou d'ammonium de molybdène, les sels de métal alcalin ou d'ammonium de tungstène, l'acide phosphomolybdique et ses sels alcalins ou d'ammonium, l'acide phosphotungstique et ses sels alcalins ou d'ammonium, les molybdosulfates et leurs mélanges, de préférence l'acide phosphomolybdique et ses sels alcalins ou d'ammonium, notamment le phosphomolybdate de sodium et le phosphomolybdate de potassium.

7. Procédé selon l'une quelconque des revendications précédentes, dans lequel au moins un agent réducteur est ajouté à la solution aqueuse obtenue à l'étape a).

8. Procédé selon l'une quelconque des revendications précédentes, dans lequel au moins un agent réducteur est ajouté à la fraction d'eaux résiduelles.

9. Procédé selon la revendication 7 ou la revendication 8, dans lequel l'agent réducteur est choisi parmi l'hydrazine, le sulfite de sodium, le bisulfite de sodium et leurs mélanges.

10. Utilisation d'un composé de formule (I) défini à la revendication 1 en tant qu'agent complexant d'un catalyseur, dans un procédé de synthèse d'un composé azoïque comprenant une étape faisant réagir un agent oxydant avec un composé hydrazo, au moins un catalyseur et au moins ledit composé de formule (I) de manière à former une solution renfermant un composé azoïque.

11. Utilisation selon la revendication 10, dans laquelle le composé de formule (I) est choisi parmi l'acide 1-hydroxy-1-(1*H*-imidazol-1-yl)éthane-1,1-diyl]diphosphonique ou un de ses sels, l'acide hydroxyméthylènediphosphonique, l'acide 1-hydroxyléthylidène-1,1-diphosphonique (HEDP) et l'acide 1-hydroxybutanediphosphonique, sous leur forme acide ou sous forme de leurs sels, de préférence le composé de formule (I) est l'acide 1-hydroxyléthylidène-1,1-diphosphonique.

**Patentansprüche**

1. Verfahren zur Synthese einer Azoverbindung, wobei das Verfahren die folgenden Schritte umfasst:

    a) das Umsetzen eines Oxidationsmittels mit einer Hydrazoverbindung, mindestens einem Katalysator und mindestens einer Verbindung der Formel (I):

    $$(R_1)(R_2)C(PO_3(R_3)_2)_2 \qquad (I)$$

    wobei

    $R_1$ und $R_2$, die identisch oder verschieden sein können, unabhängig voneinander aus einem Wasserstoffatom, einer gesättigten oder ungesättigten, linearen, verzweigten oder cyclischen, gegebenenfalls substituierten Kohlenwasserstoffkette, -OH und einem -O-Alkyl ausgewählt sind, wobei "Alkyl" eine gesättigte, gegebenenfalls substituierte, lineare oder verzweigte Kohlenwasserstoffkette darstellt, die 1 bis 6 Kohlenstoffatome umfasst; und
    $R_3$ aus einem Wasserstoffatom und Metall- oder Ammoniumionen ausgewählt ist;
    sodass eine Lösung gebildet wird, die eine Azoverbindung enthält;

    b) das Isolieren der gesamten in Schritt a) erhaltenen Reaktionsmischung oder eines Teils davon;
    c) das Trennen der isolierten Reaktionsmischung in eine die Azoverbindung enthaltende Fraktion und eine Fraktion von wässrigen Restflüssigkeiten und
    d) das Isolieren und gegebenenfalls Waschen und Trocknen der erhaltenen Azoverbindung.

2. Verfahren nach Anspruch 1, wobei die Verbindung der Formel (I) aus [1-Hydroxy-1-(1H-imidazol-1-yl)ethan-1,1-diyl]diphosphonsäure oder einem ihrer Salze, Hydroxymethylendiphosphonsäure, 1-Hydroxylethyliden-1,1-diphosphonsäure (HEDP) und 1-Hydroxybutandiphosphonsäure in ihrer Säureform oder in Form ihrer Salze ausgewählt ist, wobei die Verbindung der Formel (I) vorzugsweise 1-Hydroxylethyliden-1,1-diphosphonsäure ist.

3. Verfahren nach Anspruch 1 oder Anspruch 2, wobei die Hydrazoverbindung aus symmetrischen Hydrazoverbindungen, die stickstoffhaltige Funktionen, insbesondere Nitril- oder Amidfunktionen, tragen, insbesondere aus 2,2'-Hydrazobisisobutyronitril, 2,2'-Hydrazobismethylbutyronitril, 1,1'-Hydrazobiscyclohexancarbonitril und 2,2'-Hydrazodicarbonamid ausgewählt ist, wobei die Hydrazoverbindung vorzugsweise 2,2'-Hydrazobisisobutyronitril ist.

4. Verfahren nach einem der vorhergehenden Ansprüche, wobei das Oxidationsmittel aus anorganischen Peroxidderivaten, vorzugsweise aus Wasserstoffperoxid, Kalium- oder Natriumpersulfat, Kalium- oder Natriummonohydrogenpersulfat, Disauerstoff, Peressigsäurederivaten ausgewählt ist, wobei es sich beim Oxidationsmittel noch mehr bevorzugt um Wasserstoffperoxid handelt.

5. Verfahren nach einem der vorhergehenden Ansprüche, wobei der Katalysator eine wasserlösliche Verbindung umfasst, die aus Salzen und Säuren auf der Basis eines katalytischen Metalls der Spalten 5 und 6 des Periodensystems der Elemente ausgewählt ist und vorzugsweise aus Molybdän und Wolfram, vorzugsweise Molybdän, ausgewählt ist.

6. Verfahren nach Anspruch 5, wobei die lösliche Verbindung aus Alkalimetall- oder Ammoniumsalzen von Molybdän, Alkalimetall- oder Ammoniumsalzen von Wolfram, Molybdatophosphorsäure und deren Alkali- oder Ammoniumsalzen, Wolframatophosphorsäure und deren Alkali- oder Ammoniumsalzen, Molybdatosulfaten und Mischungen davon, vorzugsweise Molybdatophosphorsäure und deren Alkali- oder Ammoniumsalzen, insbesondere Natriummolybdatophosphat und Kaliummolybdatophosphat, ausgewählt ist.

7. Verfahren nach einem der vorhergehenden Ansprüche, wobei mindestens ein Reduktionsmittel zu der in Schritt a) erhaltenen wässrigen Lösung gegeben wird.

8. Verfahren nach einem der vorhergehenden Ansprüche, wobei mindestens ein Reduktionsmittel zu der Fraktion von wässrigen Restflüssigkeiten gegeben wird.

9. Verfahren nach Anspruch 7 oder Anspruch 8, wobei das Reduktionsmittel aus Hydrazin, Natriumsulfit, Natriumhydrogensulfit und deren Mischungen ausgewählt ist.

10. Verwendung einer Verbindung der Formel (I) gemäß der Definition in Anspruch 1 als Komplexbildner eines Katalysators in einem Verfahren zur Synthese einer Azoverbindung, das einen Schritt des Umsetzens eines Oxidationsmittels mit einer Hydrazoverbindung, mindestens einem Katalysator und mindestens einer Verbindung der Formel (I) umfasst, sodass eine Lösung gebildet wird, die eine Azoverbindung enthält.

11. Verwendung nach Anspruch 10, wobei die Verbindung der Formel (I) aus [1-Hydroxy-1-(1H-imidazol-1-yl)ethan-1,1-diyl]diphosphonsäure oder einem ihrer Salze, Hydroxymethylendiphosphonsäure, 1-Hydroxylethyliden-1,1-diphosphonsäure (HEDP) und 1-Hydroxybutandiphosphonsäure in ihrer Säureform oder in Form ihrer Salze ausgewählt ist, wobei die Verbindung der Formel (I) vorzugsweise 1-Hydroxylethyliden-1,1-diphosphonsäure ist.

**Claims**

1. Process for synthesizing an azo compound, said process comprising the steps of:

   a) reacting an oxidizing agent with a hydrazo compound, at least one catalyst and at least one compound of formula (I) :

   $$(R_1)(R_2)C(PO_3(R_3)_2)_2 \qquad (I)$$

   in which

   $R_1$ and $R_2$, which may be identical or different, are chosen independently of each other from a hydrogen atom, a saturated or unsaturated, linear, branched or cyclic, optionally substituted hydrocarbon chain, -OH and -O-alkyl, where "alkyl" represents a saturated, optionally substituted, linear or branched hydrocarbon chain comprising from 1 to 6 carbon atoms; and
   $R_3$ is chosen from a hydrogen atom and metal or ammonium ions;
   so as to form a solution containing an azo compound;

   b) recovering all or part of the reaction mixture obtained in step a);
   c) separating the reaction mixture recovered into a fraction containing the azo compound and a fraction of residual aqueous liquors; and
   d) recovering, and optionally washing and drying, the azo compound obtained.

2. Process according to Claim 1, wherein the compound of formula (I) is chosen from [1-hydroxy-1-(1H-imidazol-1-yl)ethane-1,1-diyl]diphosphonic acid or one of its salts, hydroxymethylenediphosphonic acid, 1-hydroxylethylidene-1,1-diphosphonic acid (HEDP) and 1-hydroxybutanediphosphonic acid, in their acid form or in the form of their salts; the compound of formula (I) is preferably 1-hydroxylethylidene-1,1-diphosphonic acid.

3. Process according to Claim 1 or Claim 2, wherein the hydrazo compound is chosen from symmetrical hydrazo compounds bearing nitrogen-based functional groups, in particular nitrile or amide functional groups, preferably from 2,2'-hydrazobisisobutyronitrile, 2,2'-hydrazobismethylbutyronitrile, 1,1'-hydrazobiscyclohexanecarbonitrile and 2,2'-hydrazodicarbonamide; the hydrazo compound is preferably 2,2'-hydrazobisisobutyronitrile.

4. Process according to any one of the preceding claims, wherein the oxidizing agent is chosen from inorganic peroxide derivatives, preferably from hydrogen peroxide, potassium or sodium persulfates, potassium or sodium monohydrogen persulfates, dioxygen, derivatives of peracetic acid; the oxidizing agent preferably more preferably is hydrogen peroxide.

5. Process according to any one of the preceding claims, wherein the catalyst comprises a water-soluble compound chosen from salts and acids based on a catalytic metal from columns 5 and 6 of the Periodic Table of the Elements and preferably chosen from molybdenum and tungsten, preferably molybdenum.

6. Process according to Claim 5, wherein the soluble compound is chosen from alkali metal or ammonium salts of molybdenum, alkali metal or ammonium salts of tungsten, phosphomolybdic acid and alkali metal or ammonium salts thereof, phosphotungstic acid and alkali metal or ammonium salts thereof, molybdosulfates and mixtures thereof, preferably phosphomolybdic acid and alkali metal or ammonium salts thereof, in particular sodium phosphomolybdate and potassium phosphomolybdate.

7. Process according to any one of the preceding claims, wherein at least one reducing agent is added to the aqueous solution obtained in step a).

8. Process according to any one of the preceding claims, wherein at least one reducing agent is added to the fraction of residual aqueous liquors.

9. Process according to Claim 7 or Claim 8, wherein the reducing agent is chosen from hydrazine, sodium sulfite, sodium bisulfite and mixtures thereof.

10. Use, as a catalyst complexing agent, of a compound of formula (I) defined in Claim 1, in a process for synthesizing an azo compound comprising a step of reacting an oxidizing agent with a hydrazo compound, at least one catalyst and at least said compound of formula (I) so as to form a solution containing an azo compound.

11. Use according to Claim 10, wherein the compound of formula (I) is chosen from [1-hydroxy-1-(1H-imidazol-1-yl)ethane-1,1-diyl]diphosphonic acid or one of its salts, hydroxymethylenediphosphonic acid, 1-hydroxylethylidene-1,1-diphosphonic acid (HEDP) and 1-hydroxybutanediphosphonic acid, in their acid form or in the form of their salts; the compound of formula (I) is preferably 1-hydroxylethylidene-1,1-diphosphonic acid.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2006067315 A
- GB 976552 A
- EP 2821393 A
- WO 2006072699 A

**Littérature non-brevet citée dans la description**

- **A. PALOMO.** *Afinidad,* 1985, vol. 42 (397), 312-314